# EUROPEAN PATENT APPLICATION

(11) **EP 4 052 715 A1**
(43) Date of publication of application: **07.09.2022**
(21) Application number: 20881317.0
(22) Date of filing: 30.10.2020
(51) Int. Cl.: A61K 35/17, A61P 35/00

(54) **COMPOSITION FOR TREATMENT AND/OR PREVENTION OF TUMOR**

(30) Priority: 31.10.2019 JP 2019199195
(71) Applicant: Fukushima Medical University, Fukushima 960-1295 (JP)
(72) Inventor: MOCHIZUKI, Kazuhiro, Fukushima-shi, Fukushima 960-1295 (JP)
(74) Representative: D Young & Co LLP
(86) International application number: PCT/JP2020/040920
(87) International publication number: WO 2021/085624

(57) **Abstract**

In one embodiment, a problem to be addressed by the present invention is to provide a novel cellular immunotherapy having a treatment and/or prevention effect against a tumor. In one embodiment, the present invention relates to a composition for use in treatment and/or prevention of a tumor in a subject comprising allogeneic CD4⁺ T cells, wherein the allogeneic CD4⁺ T cells have (a) MHC class II molecules all or part of which are different from MHC class II molecules of the subject, and contain (b) cells activated by *ex vivo* coculture with antigen-presenting cells derived from the subject or an individual having MHC class II molecules wholly identical or partially identical with the MHC class II molecules of the subject.

## Description

### Technical Field

In one embodiment, the present invention relates to the following: a composition for use in the treatment and/or prevention of a tumor in a subject comprising an allogeneic CD4⁺ T cell; a method of producing the composition; or a method of treatment and/or prevention of a tumor in a subject.

### Background Art

Immuno/cellular therapies proposed in the past 10 years are recognized as promising therapies for intractable cancers, and some of the therapies are already available clinically. However, for example, efficacies of immune checkpoint blockade therapies for various cancers are approximately 15%, and such therapies have achieved no effect for a majority of the remaining patients (Non-Patent Literature 1). For example, a chimeric antigen receptor (CAR) T cell therapy is an epoch-making method, but applications of the therapy has not been extended to neoplasms other than B cell hematologic neoplasms, due to limited promising target antigens for the therapy. In addition, the therapy is inefficacious against tumor cells which lack a target antigen for a CAR-T cell. Furthermore, the therapy is very expensive personalized medicine, and has not come to be generally and widely utilized.

An ultimate goal of a cancer therapy is to suppress a cancer and, at the same time, prevent permanent recurrences of the cancer. To achieve the object, it is ideal to induce potent antitumor immunity in a host itself.

Vaccination with cancer associated multiple peptides (CAMPs) or vaccination with autologous dendritic cells pulsed with CAMPs is a theoretical method for achieving the object. However, clinical trials up to now have demonstrated that these cancer vaccinations utilizing an autologous immune system achieve a limited efficacy (Non-Patent Literatures 2 and 3).

In recent years, there have been a plurality of reports on methods of inducing antitumor immunity in a host using allogeneic CD4⁺ T cells. Examples of such methods include a method using nonspecific allogeneic CD4⁺ T cells inactivated with mitomycin C and a method using allogeneic CD4⁺ T cells nonspecifically activated with CD3/CD28 antibodies. However, any of these methods fails to obtain an effect by a single administration, and requires multiple administration to activate the immunity of a host. In addition, any of these methods activates the host immunity nonspecifically, and thus, needs to be used in combination with a cancer vaccine therapy to afford sufficient tumor specificity. Accordingly, such methods are not significantly different from a conventional cancer vaccine therapy in principle. (Non-Patent Literatures 4 to 6)

As mentioned above, cellular/immune therapies are attracting attention as therapies for intractable cancers, but efficacies of existing therapies are limited. Therefore, there is a need for developing a novel therapy.

### Citation List

### Non-Patent Literature

Non-Patent Literature 1: Pitt JM. et al. Resistance Mechanisms to Immune- Checkpoint Blockade in Cancer: Tumor-Intrinsic and -Extrinsic Factors. Immunity, 2016, 44:1255-69.
Non-Patent Literature 2: Bezu L. et al., Trial watch: Peptide-based vaccines in anticancer therapy., Oncoimmunol., 2018, 7: e1511506
Non-Patent Literature 3: Rosenberg SA.et al. Cancer Immunotherapy: Moving Beyond Current Vaccines Nat Med., 2004, Sep; 10(9):909-15.
Non-Patent Literature 4: Tang Y. et al. A tritherapy combination of inactivated allogeneic leukocytes infusion and cell vaccine with cyclophosphamide in a sequential regimen enhances antitumor immunity. J Chin Med Assoc., 2018, 81(4):316-323.
Non-Patent Literature 5: Har-Noy M. et al. Allogeneic CD3/CD28 cross-linked Th1 memory cells provide potent adjuvant effects for active immunotherapy of leukemia/lymphoma. Leuk Res, 2009, 33(4):525-38.
Non-Patent Literature 6: Janikashvili N. et al. Allogeneic effector/memory Th-1 cells impair FoxP3_ regulatory T lymphocytes and synergize with chaperone-rich cell lysate vaccine to treat leukemia. Blood, 2011, 117(5):1555-64.

### Summary of Invention

### Technical Problem

In one embodiment, a problem to be addressed by the present invention is to provide a novel cellular immunotherapy having a treatment and/or prevention effect on a tumor.

### Solution to Problem

The present inventor has completed the present invention through the discovery that an allogeneic CD4⁺ T cell can induce antitumor immunity in a subject, and achieve an effect on a tumor, wherein the allogeneic CD4⁺ T cell (a) has MHC class II molecules all or part of which are different from the MHC class II molecules of the subject, and (b) has been cocultured *ex vivo* with an antigen-presenting cell derived from the subject or an individual having MHC class II molecules wholly identical or partially identical with the MHC class II molecules of the subject.

The present invention encompasses the following embodiments.
(1) A composition for use in treatment and/or prevention of a tumor in a subject comprising an allogeneic CD4⁺ T cell,
   wherein the allogeneic CD4⁺ T cell has (a) MHC class II molecules all or part of which are different from MHC class II molecules of the subject, and contains (b) a cell cocultured *ex vivo* with an antigen-presenting cell derived from the subject or an individual having MHC class II molecules wholly identical or partially identical with the MHC class II molecules of the subject.
(2) The composition according to (1), wherein the antigen-presenting cell is a dendritic cell.
(3) The composition according to (1) or (2), for use in intratumoral administration.
(4) A method of producing the composition according to any one of (1) to (3), comprising the step of coculturing an allogeneic CD4⁺ T cell *ex vivo* with an antigen-presenting cell derived from the subject or an individual having MHC class II molecules wholly identical or partially identical with the MHC class II molecules of the subject.

The present description encompasses the contents disclosed in Japanese Patent Application No. 2019-199195, on which the priority of the present application is based.

### Advantageous Effects of Invention

The composition including an allogeneic CD4⁺ T cell according to the present invention can achieve a treatment and/or prevention effect on a tumor.

### Brief Description of Drawings

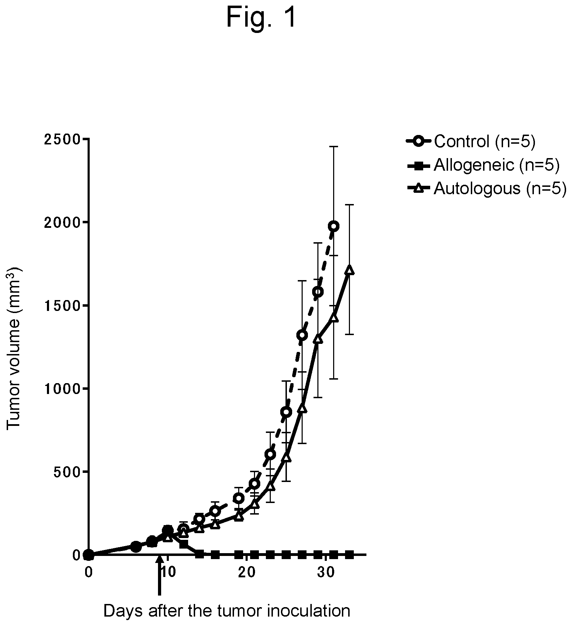

[Figure 1] Figure 1 shows tumor volumes when CD4⁺ T cells isolated from BALB/c mice were cocultured with dendritic cells derived from C57BL/6 mice, and then the obtained allogeneic CD4⁺ T cells were administered intratumorally to C57BL/6 mice which had been subcutaneously inoculated with a tumor (B16F1, 0.5 × 10⁶ cells) (n = 5). The graph also includes the results of not administering the CD4⁺ T cells, as a control (n = 5), and the results of administering CD4⁺ T cells isolated from C57BL/6 mice (autologous) (n = 5). The arrow indicates ninth day after the tumor inoculation when the CD4⁺ T cells were administered (the same applies to the following drawings).

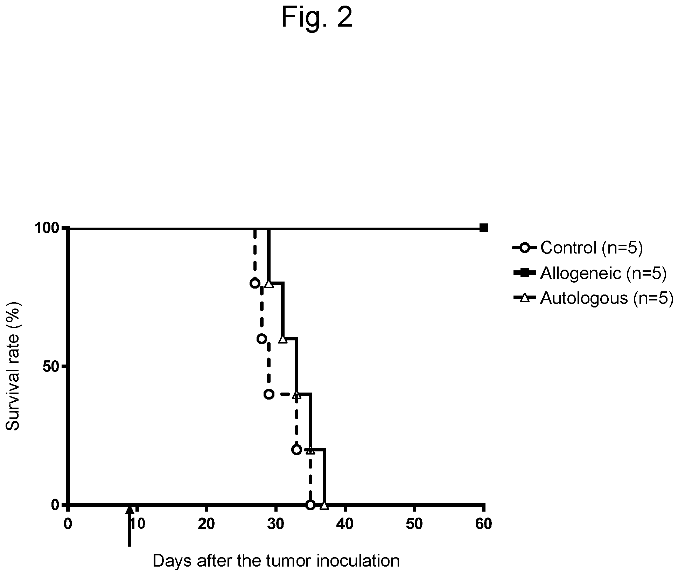

[Figure 2] Figure 2 shows survival rates when CD4⁺ T cells isolated from BALB/c mice were cocultured with dendritic cells derived from C57BL/6 mice, and then the obtained allogeneic CD4⁺ T cells were administered intratumorally to C57BL/6 mice which had been subcutaneously inoculated with a tumor (B16F1, 0.5 × 10⁶ cells) (n = 5). The graph also includes the results of not administering the CD4⁺ T cells, as a control (n = 5), and the results of administering CD4⁺ T cells isolated from C57BL/6 mice (autologous) (n = 5).

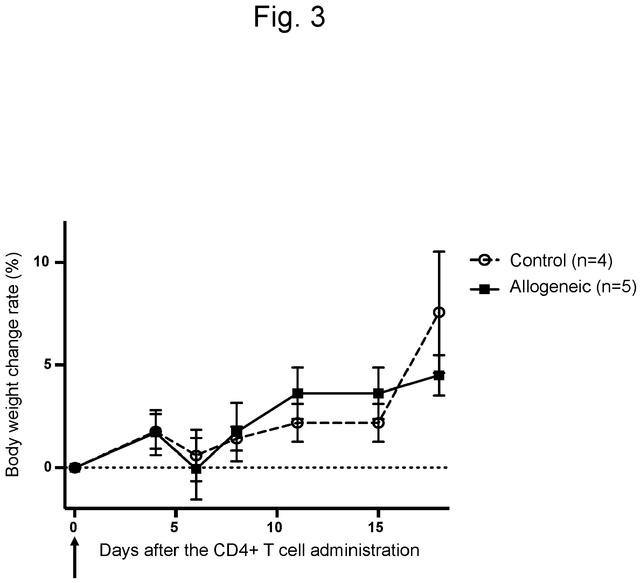

[Figure 3] Figure 3 shows body weight change rates when CD4⁺ T cells isolated from BALB/c mice were cocultured with dendritic cells derived from C57BL/6 mice, and then the obtained allogeneic CD4⁺ T cells were administered intratumorally to C57BL/6 mice which had been subcutaneously inoculated with a tumor (B16F, 0.5 × 10⁶ cells) (n = 5). The graph also includes the results of not administering the CD4⁺ T cell, as a control (n = 4).

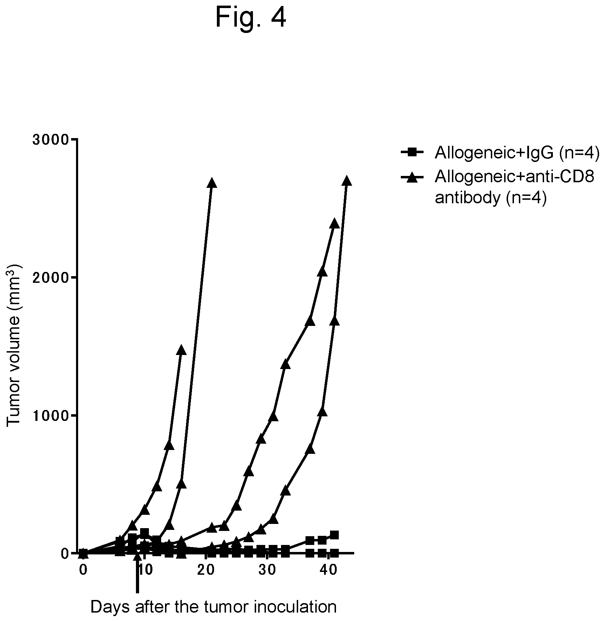

[Figure 4] Figure 4 shows tumor volumes when C57BL/6 mice were subcutaneously inoculated with 1.0 × 10⁶ cells of B16F1 on day 0, host CD8⁺ cells were depleted from the C57BL/6 mice by an anti-CD8 antibody on day 8, and then, allogeneic CD4⁺ T cells (derived from BALB/c mice) which had been cocultured with dendritic cells derived from C57BL/6 mice were administered intratumorally to the C57BL/6 mice (n = 4). The graph also includes the results of administering a control IgG antibody which does not deplete the host CD8⁺ cells, as a control (n =4).

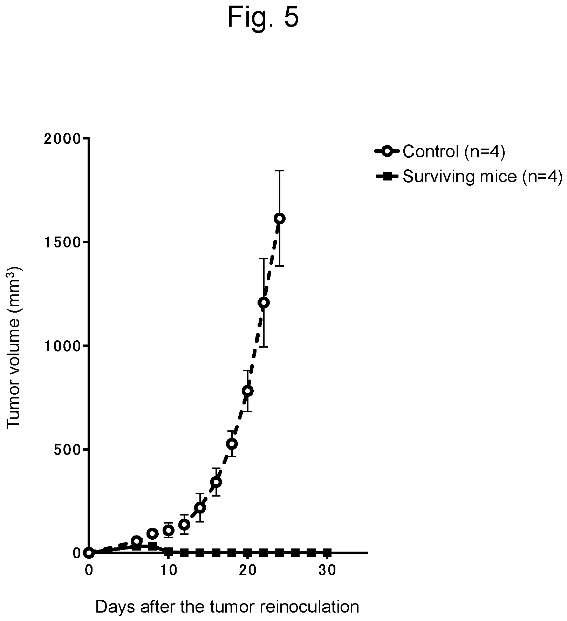

[Figure 5] Figure 5 shows tumor volumes when allogeneic CD4⁺ T cells (derived from BALB/c mice) cocultured with dendritic cells derived from C57BL/6 mice were administered to C57BL/6 mice which had been subcutaneously inoculated with 0.5 × 10⁶ cells of B16F1 for the first time, and after six months, the mice survived were inoculated with a tumor (subcutaneously inoculated with 2.5 × 10⁶ cells of B16F1) for the second time (n = 4). As a control, the graph also includes the results of inoculating naive mice (which had not been inoculated with a tumor and had not received immunotherapy in the past) with a tumor (n = 4).

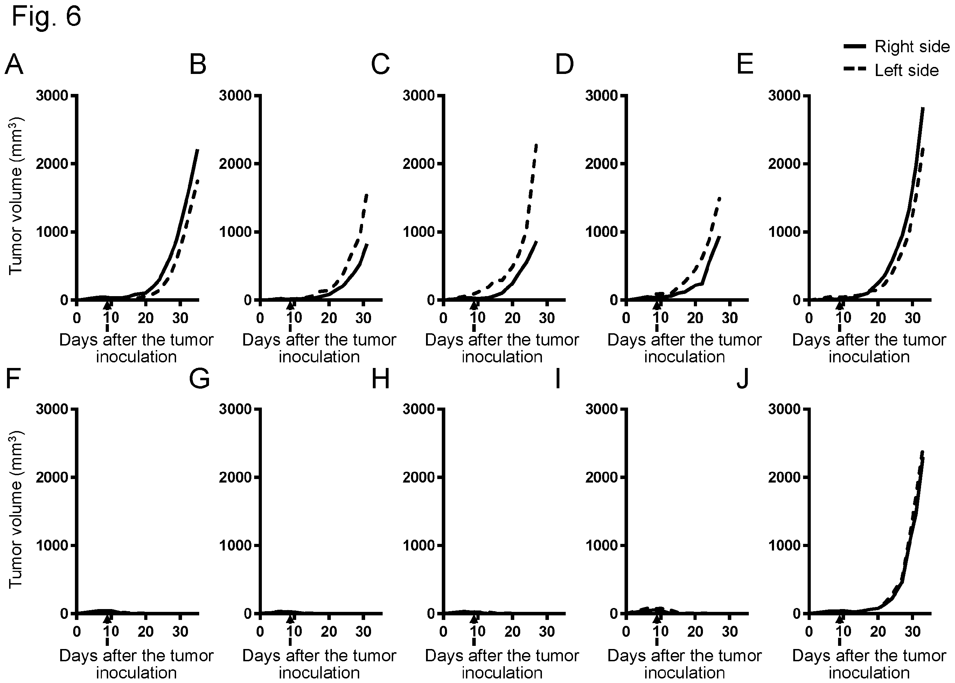

[Figure 6] Figure 6 shows a tumor volume in the right or left side abdomen of each mouse when the right side abdomen and left side abdomen of C57BL/6 mice were subcutaneously inoculated with B16F1 (1.0 × 10⁶ cells for each side); and then, allogeneic CD4⁺ T cells (derived from BALB/c mice) which had been cocultured with dendritic cells derived from C57BL/6 mice were administered only to the tumor in the left side abdomen (n = 5 for each; A to E show the results of each control mouse, and F to J show the results of each mouse which received the allogeneic CD4⁺ T cells).

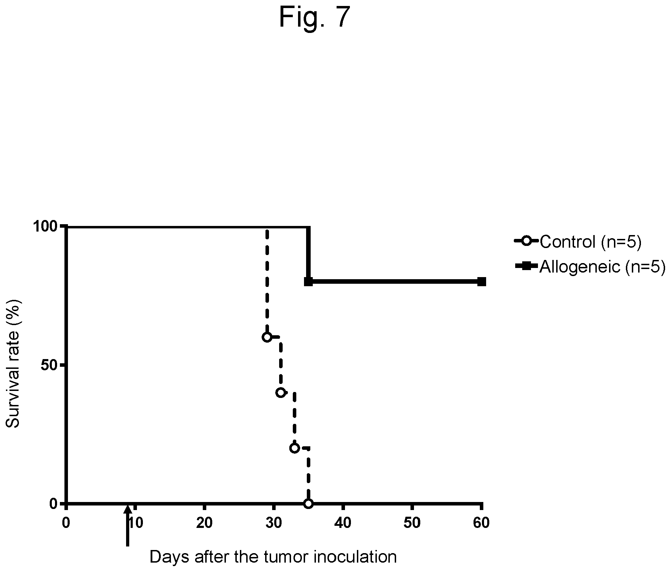

[Figure 7] Figure 7 shows survival rates of mice when the right side abdomen and left side abdomen of C57BL/6 mice were subcutaneously inoculated with B16F1 (1.0 × 10⁶ cells for each side); and then, allogeneic CD4⁺ T cells (derived from BALB/c mice) which had been cocultured with dendritic cells derived from C57BL/6 mice were administered only to the tumor in the left side abdomen (n = 5 for each).

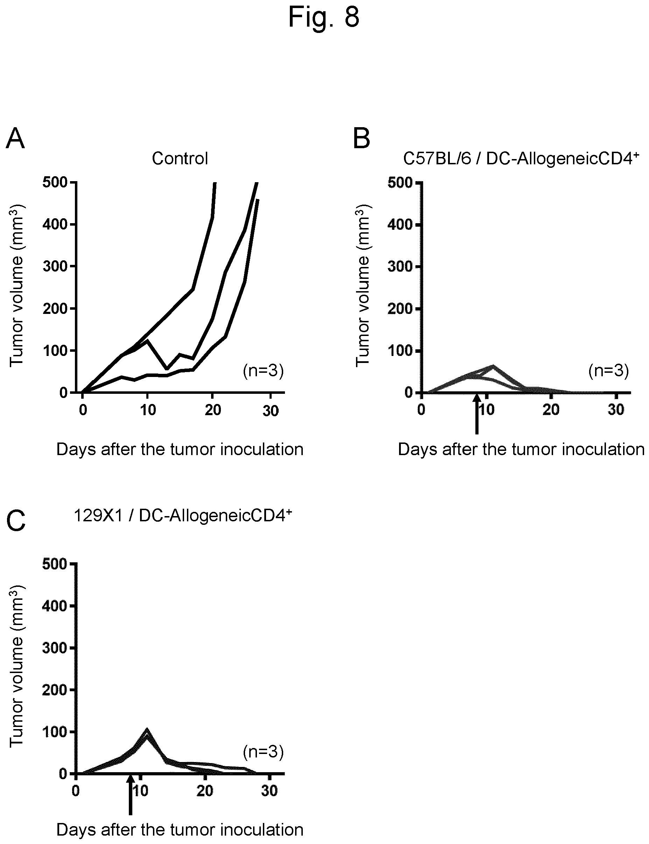

[Figure 8] Figure 8 shows tumor volumes when CD4⁺ T cells isolated from BALB/c mice were cocultured with dendritic cells derived from C57BL/6 or 129X1 mice; and the obtained allogeneic CD4⁺ T cells were administered intratumorally to C57BL/6 mice which had been inoculated with a tumor (B16F1, 1.0 × 10⁶ cells). Figure 8A shows the results of not administering the CD4⁺ T cells, as a control (n = 3). Figure 8B shows the results of administering the allogeneic CD4⁺ T cells which had been cocultured with the dendritic cells derived from C57BL/6 mice (n = 3). Figure 8C shows the results of administering the allogeneic CD4⁺ T cells which had been cocultured with the dendritic cells derived from 129X1 mice (n = 3).

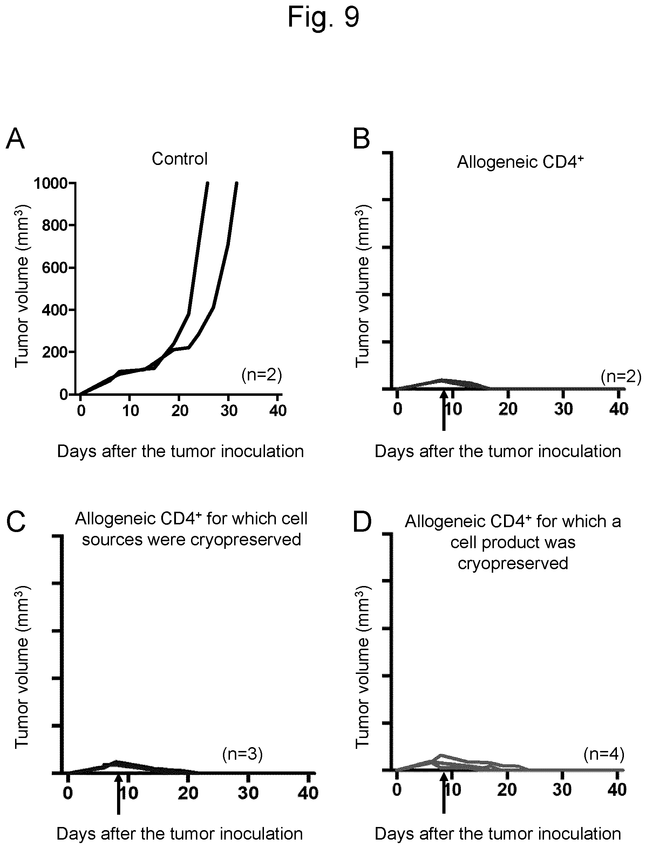

[Figure 9] Figure 9 shows tumor volumes when CD4⁺ T cells isolated from BALB/c mice were cocultured with dendritic cells derived from C57BL/6 mice, and then the obtained allogeneic CD4⁺ T cells were administered intratumorally to C57BL/6 mice which had been inoculated with a tumor (B16F1, 1.0 × 10⁶ cells). In the drawing, "cell sources were cryopreserved" means that the cell sources were frozen at -80°C at the stage of being c-kit-positive cells isolated from the bone marrow of C57BL/6 mice or at the stage of being CD4⁺ T cells isolated from BALB/c mice, and were thawed when used for culture. Further, "a cell product was cryopreserved" means that CD4⁺ T cells which had been cocultured with dendritic cells derived from C57BL/6 mice were prepared and then cryopreserved at -80°C, and thawed before being administered. Figure 9A shows the results of not administering the CD4⁺ T cells, as a control (n = 2). Figure 9B shows the results of administering allogeneic CD4⁺ T cells obtained without cryopreserving cell sources and a cell product (n = 2). Figure 9C shows the results of administering the allogeneic CD4⁺ T cells for which cell sources were frozen (n = 3). Figure 9D shows the results of administering the allogeneic CD4⁺ T cells for which a cell product was frozen (n = 4).

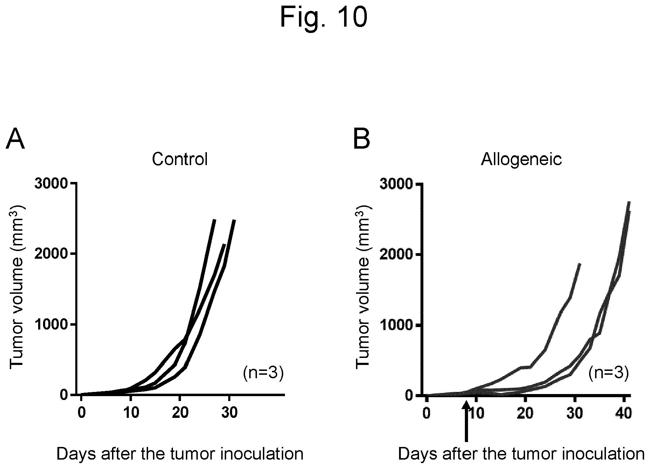

[Figure 10] Figure 10 shows tumor volumes when CD4⁺ T cells isolated from C57BL/6 mice were cocultured with dendritic cells derived from BALB/c mice, and then the obtained allogeneic CD4⁺ T cells were administered intratumorally to BALB/c mice which had been inoculated with a tumor (Colon-26). Figure 10A shows the results of not administering the CD4⁺ T cells, as a control (n = 3). Figure 10B shows the results of administering the allogeneic CD4⁺ T cells (n = 3).

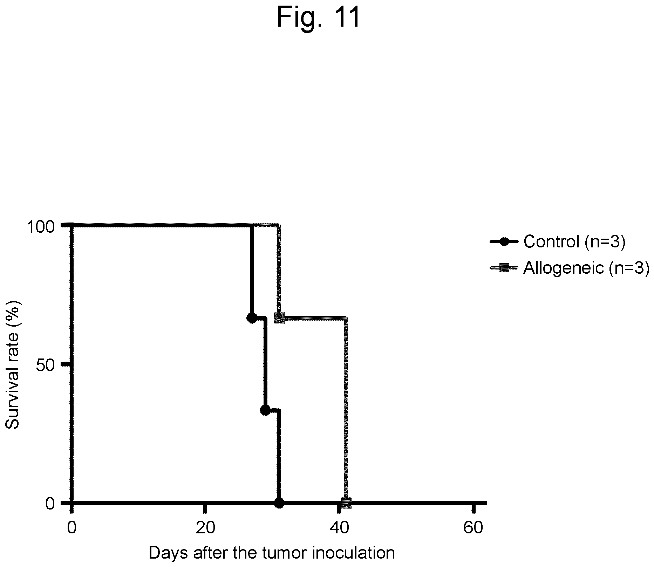

[Figure 11] Figure 11 shows survival rates of BALB/c mice when CD4⁺ T cells isolated from C57BL/6 mice were cocultured with dendritic cells derived from BALB/c mice, and then the obtained allogeneic CD4⁺ T cells were administered intratumorally to BALB/c mice which had been inoculated with a tumor (Colon-26, 5 × 10⁴ cells) (n = 3). The graph also includes the results of not administering the CD4⁺ T cells, as a control (n = 3).

### Description of Embodiments

In one aspect, the present invention relates to a composition for use in treatment and/or prevention of a tumor in a subject comprising an allogeneic CD4⁺ T cell(s). The allogeneic CD4⁺ T cell(s) has (a) MHC class II molecules all or part of which are different from MHC class II molecules of the subject. In addition, the allogeneic CD4⁺ T cell(s) contains (b) a cell(s) cocultured *ex vivo* with an antigen-presenting cell(s) derived from the subject or an individual having MHC class II molecules wholly identical or partially identical with the MHC class II molecules of the subject. The allogeneic CD4⁺ T cell(s) may be composed (consisted) substantially of the above-mentioned cell(s), or may be composed (consisted) only of the above-mentioned cell(s).

The species of the "subject" as used herein, include, but are not limited to, mammals, for example, primates such as humans and rhesus monkeys; laboratory animals such as rats, mice, and brown rats; livestock animals such as pigs, cattle, horses, sheep, and goats; pet animals such as dogs and cats; and the like. The subject is preferably a human. The subject may be a subject that has a tumor, or is suspected of having a tumor. Whether a subject has a tumor, and whether a subject is suspected of having a tumor can be checked by a conventional method. In one embodiment, the subject can be an individual that had a tumor, which was treated (that is, which has the risk of recurrence).

The term "tumor" means a benign tumor and a malignant tumor, and, as used herein, particularly means a malignant tumor (cancer). Types of the "tumor" include, but are not limited to, an adenocarcinoma, squamous cell carcinoma, small cell cancer, large cell cancer, and the like. Specifically, types of the tumor include: malignant melanoma; oral cancer; laryngeal cancer; pharyngeal cancer; thyroid cancer; lung cancer; breast cancer; esophageal cancer; stomach cancer; large bowel cancer (encompassing colonic cancer and rectal cancer); small intestinal cancer; bladder cancer; prostate cancer; testicular cancer; endometrial cancer; cervical cancer; ovarian cancer; stomach cancer; kidney cancer; pancreas cancer; biliary tract cancer; gallbladder cancer; brain tumors; osteosarcoma; childhood tumors including neuroblastoma; leukemia; lymphomas; and the like. The tumor is preferably a solid tumor because a solid tumor enables the below-mentioned intratumoral administration. The tumor may be an intractable tumor that is difficult to treat by another therapy. Further, the tumor may be an intractable tumor having a metastatic lesion.

As used herein, the term "allogeneic" is also expressed as "being a separate individual of the same species" or "being a different individual of the same species", and is intended for a different individual of the same species, excluding monozygotic twins.

As used herein, the term "autologous" is also expressed as "self', and is intended for the same individual.

The composition according to the present invention preferably comprises allogeneic CD4⁺ T cells as an effective component. Method of obtaining the allogeneic CD4⁺ T cells are not limited, and the allogeneic CD4⁺ T cells can be obtained, for example, using a method known to a person skilled in the art. For example, allogeneic CD4⁺ T cells can be obtained from an individual allogeneic to a subject as follows: lymphocyte fractions are obtained from blood by apheresis, and allogeneic CD4⁺ T cells are obtained from the lymphocyte fractions, for example, using microbead-conjugated anti-CD4 antibodies, or by flow cytometry using anti-CD4 antibodies. Alternatively, allogeneic CD4⁺ T cells can be obtained by removing cells other than CD4⁺ T cells from the lymphocyte fractions.

Herein, the allogeneic CD4⁺ T cell has MHC (major histocompatibility complex) class II molecules all or part of which are different from MHC class II molecules of a subject. As used herein, the term "MHC" is a group of antigens on the surface of a cell which induces graft rejection in transplantation immunity, and is known as a human leukocyte antigen (HLA) in a human, and as an H-2 antigen (histocompatibility-2) in a mouse. The MHC includes a class I molecule and a class II molecule. The MHC class II molecule is involved in antigen presentation by an antigen-presenting cell, and activates a CD4⁺ T cell. In contrast, the MHC class I molecule activates a CD8⁺ T cell. From the results of allogeneic hematopoietic stem cell transplantation between individuals having different HLAs, it is known that, for example, a difference in any one of HLA-DR, HLA-DP, and HLA-DQ belonging to MHC class II molecules increases a risk ratio of onset of complications based on an alloimmune reaction after transplantation (for example, an acute graft-versus-host disease), in a human. This indicates that a difference in at least one of MHC class II molecules can activate allogeneic immunity. Accordingly, an effect of the present invention can be obtained when using an allogeneic CD4⁺ T cell which has MHC class II molecules at least one of which is different from those of a subject. Therefore, the phrase "MHC class II molecules are different", as used herein includes that, for example, at least one of HLA-DR, HLA-DP, and HLA-DQ is different in a human. For example, the phrase means that two of these, preferably all the three are different.

An allogeneic CD4⁺ T cell is activated by MHC class II molecules, but does not respond to MHC class I molecules (for example, the HLA-A, HLA-B, and HLA-C in a human). Accordingly, MHC class I molecules of the allogeneic CD4⁺ T cell in the present invention may be identical with or may not be identical with, i.e., may be wholly identical with, partially identical with, or completely different from MHC class I molecules of a subject.

Combinations of MHCs in a haplotype are diverse, and it is said that there are tens of thousands of such combinations for human HLAs. Thus, MHCs of different individuals of the same species are usually not identical. On the other hand, a child inherits MHC genes by half from each parent, and thus, MHCs are wholly identical with a probability of 1/4 between brothers and sisters. Further, MHCs are wholly identical between monozygotic twins. In this regard, whether MHCs are identical can be examined by a method known to a person skilled in the art. Example of such test methods include a serologic test (in which MHC typing is performed in accordance with reactivity between an antiserum with known MHC specificity and lymphocytes) and a DNA type test (for example, a PCR-SSO method, PCR-SSP method, PCR-SBT method, typing by next-generation sequencing, or the like).

The allogeneic CD4⁺ T cell is cocultured *ex vivo* with an antigen-presenting cell derived from a subject or an individual having MHC class II molecules wholly identical or partially identical with MHC class II molecules of the subject. The coculture can activate an allogeneic CD4⁺ T cell clone that reacts with MHC class II molecules of a subject, more selectively. As used herein, the phrase "individual having MHC class II molecules wholly identical or partially identical with MHC class II molecules of a subject" encompasses an individual having MHC class II molecules wholly the same as a subject, and in addition, an individual having partially different group of MHC class II molecules, and an individual having MHC class II molecules substantially the same as a subject. Here, the phrase "individual having MHC class II molecules substantially the same as a subject" is intended to permit any slight difference in the amino acids (for example, one to five amino acids, one to four amino acids, one to three amino acids, one to two amino acids, or one amino acid) between MHC class II molecules as long as the *ex vivo* coculture with the antigen-presenting cell can initiate immune response of the CD4⁺ T cells to subject-derived antigens (MHC class II molecules).

Herein, the "individual having MHC class II molecules wholly identical or partially identical with MHC class II molecules of a subject", which can provide, *ex vivo,* the subject with antigen-presenting cells to be cocultured with the allogeneic CD4⁺ T cells or a cell source for the antigen-presenting cells, encompasses, for example monozygotic twins of a subject, a subject's blood relative (for example, brother or sister) having identical MHC class II molecules, and in addition, a different individual of the same species who has identical or partially identical MHC class II molecules coincidentally. Coculturing allogeneic CD4⁺ T cells and antigen-presenting cells obtained from the individual having MHC class II molecules wholly identical or partially identical with the MHC class II molecules of the subject, and administering the obtained allogeneic CD4⁺ T cells to the subject, elicit a strong immune response to subject-derived antigens (MHC class II molecules), leading to initiation of an inflammatory reaction, which is accompanied by induction of antitumor immunity in the subject.

As used herein, the phrase *"ex vivo* coculture" is intended to mean a culture in an artificial *in vitro* environment. Conditions of the *ex vivo* coculture of the CD4⁺ T cells and the antigen-presenting cells are not limited, and can be usual culture conditions. For example, a culture can be performed using a commercially available culture medium (for example, DMEM, MEM, BME, RPMI 1640, F-10, F-12, DMEM-F12, α-MEM, IMDM, McCoy's 5A culture medium, or mTeSR1 culture medium) or a prepared culture medium. Any kind of additive (for example, at least one of a serum or serum substitute, an immune-stimulating factor such as IL-2, a stimulus amplifier such as a Notch ligand, L-glutamine, a nonessential amino acid, 2-mercaptoethanol, an antibiotic such as penicillin or streptomycin, and a growth factor such as a basic fibroblast growth factor) can be used, for example, can be added to the culture medium, or can be solid-phased to a culture plate. A culture temperature can be approximately 30°C to approximately 40°C, approximately 35°C to approximately 39°C, approximately 36°C to approximately 38°C, or approximately 37°C. A culture may be performed in the presence of CO₂, and CO2 concentration may be approximately 2% to approximately 10%, approximately 4% to approximately 6%, or approximately 5%. A culture period may be, for example, 12 hours to 2 weeks, 1 day to 1 week, 2 days to 4 days, or approximately 3 days. In addition, a ratio of the CD4⁺ T cells and the antigen-presenting cells in a coculture is not limited, and for example, the ratio of the CD4⁺ T cells to the antigen-presenting cells may be 0.25 to 16, 0.5 to 8, 1 to 6, or 2 to 4.

The CD4⁺ T cells can be cryopreserved before and/or after the *ex vivo* coculture with the antigen-presenting cells. A cryopreservation method is known to a person skilled in the art. For example, cells are separated by centrifugation or the like, and to the cells, a culture medium or the like containing a cell preservation solution such as DMSO is added. The resulting mixture is cooled with liquid nitrogen or the like so that the cells can be preserved. As the cell preservation solution, a commercially available cell preservation solution can be used.

After the coculture, the CD4⁺ T cells may be isolated from the antigen-presenting cells. Alternatively, the CD4⁺ T cells may be used together with the antigen-presenting cells without isolating the CD4⁺ T cells from the coculture, because the CD4⁺ T cells grow, but the antigen-presenting cells hardly grow during the coculture.

The CD4⁺ T cells after the coculture can contain non-alloreactive CD4⁺ T cells that have not been activated, in addition to alloreactive CD4⁺ T cells that have been activated by antigen-presenting cells. The CD4⁺ T cells after the coculture with inactivated CD4⁺ T cells may be used, or only activated CD4⁺ T cells may be isolated and used. When the CD4⁺ T cells after the coculture contain both activated and inactivated cells, a portion of the activated cells is not limited, but may be, for example, 3 to 90%, 5 to 80%, 10 to 70%, 15 to 60%, or 20 to 50%. Methods for isolating activated CD4⁺ T cells are not limited. For example, activated CD4⁺ T cells can be isolated by sorting based on flow cytometry using an activation marker such as CD44 as an indicator.

It is also possible to isolate alloreactive CD4⁺ T cells that have been activated by a coculture with antigen-presenting cells, produce iPS cells from the CD4⁺ T cells, and then, regenerate CD4⁺ T cells from the iPS cells and use them. All of the iPS cell-derived regenerated CD4⁺ T cells, in which reconstitution of a T cell receptor gene has been completed, have the same T cell receptor specificity as the original CD4⁺ T cells that have been activated to have alloreactivity. Accordingly, administering the regenerated CD4⁺ T cells to a subject can elicit an immune response to subject-derived antigens (MHC class II molecules), leading to initiation of an inflammatory reaction, which is accompanied by induction of antitumor immunity in the subject.

The antigen-presenting cell includes, for example, a dendritic cell, a monocyte, a macrophage, and a B cell, and are preferably a dendritic cell.

Methods of obtaining antigen-presenting cells and cell sources are known to a person skilled in the art, and are not limited. For example, dendritic cells can be obtained, as described in the below-mentioned EXAMPLE, by isolating hematopoietic stem cells on the basis of a marker (for example, c-kit-positive for a mouse, or CD34-positive for a human) from bone marrow blood obtained through harvest of bone marrow, and culturing the hematopoietic stem cells under specific conditions. Alternatively, dendritic cells may be obtained by isolating hematopoietic stem cells from umbilical cord blood, and culturing the hematopoietic stem cells under specific conditions. Alternatively, it is also possible to obtain peripheral blood mononuclear cells (PBMCs) from a peripheral whole blood by density gradient centrifugation using Ficoll-Hypaque, Ficoll-Conray, or the like as a specific gravity liquid, and mature or isolate dendritic cells from the PBMCs.

Dendritic cells can be cryopreserved at each of the above-mentioned preparation stages, for example, before or after maturing. A cryopreservation method is known to a person skilled in the art. For example, cells are separated by centrifugation or the like, and to the cells, a culture medium or the like containing a cell preservation solution such as DMSO is added. The resulting mixture is cooled with liquid nitrogen or the like so that the cells can be preserved.

The composition according to the present invention may comprise a component other than the CD4⁺ T cells described herein, or may be composed (consisted) substantially of the CD4⁺ T cells described herein. The composition according to the present invention may be a pharmaceutical composition.

An amount of the CD4⁺ T cells contained in the composition according to the present invention (for example, an effective amount for treatment and/or prevention) can be suitably determined by a person skilled in the art, considering various factors such as gender, body weight, and age of a subject and courses and symptoms of a disease in the subject. For example, the amount of the CD4⁺ T cells contained in the composition according to the present invention can be adjusted to, but is not limited to, for example, approximately 1 × 10⁴ cells/kg to 1 × 10¹⁰ cells/kg, 1 × 10⁵ cells/kg to 1 × 10⁹ cells/kg, or 1 × 10⁶ cells/kg to 1 × 10⁸ cells/kg, per 1 kg of a body weight of a subject to whom the composition is administered.

The composition according to the present invention may comprise another component, for example, at least one of sterile water, physiological saline, a buffer, a stabilizer, a cell stabilizer, and a protein such as albumin, in addition to the CD4⁺ T cells.

The composition according to the present invention can be prepared by a conventional method. For the preparation, for example, a method described in Remington's Pharmaceutical Sciences (Merck Publishing Co., Easton, Pa.) can be referred to.

Dosage forms are not limited, and are suitably selected as desired. The composition can be administered, for example, in the form of an injection or a drop.

The amount of the CD4⁺ T cells described herein, or the dose (for example, the effective amount for treatment and/or prevention), dosage interval, and dosing period of the composition described herein can be suitably determined by a person skilled in the art, considering various factors such as the gender, body weight, and age of a subject and courses and symptoms of a disease in the subject. For example, the dose of the CD4⁺ T cells may be approximately 1 × 10⁴ cells/kg to 1 × 10¹⁰ cells/kg, 1 × 10⁵ cells/kg to 1 × 10⁹ cells/kg, or 1 × 10⁶ cells/kg to 1 × 10⁸ cells/kg. In addition, frequency of administration may be, without limitation, three times a day, twice a day, once a day, every two days, every three days, once a week, every two weeks, once a month, or the like. In addition, dosing period may be, without limitation, one day, two days, three days, one week, two weeks, one month, half a year, one year, or longer.

Herein, dosage forms of the composition are not limited, and may be, for example, parenteral administration. Specific examples of parenteral administration include intravenous administration, intraarterial administration, subcutaneous administration, intradermal administration, tracheal/intrabronchial administration, intrathoracic administration, intraperitoneal administration, rectal administration, intramuscular administration, peridural administration, regional lymph node administration, and intratumoral administration. The composition according to the present invention may be prepared for these dosage forms. For example, the composition may be prepared so as to satisfy at least one of sterile, low endotoxin, and virus-free, for example, satisfy all the three conditions. In one embodiment, the composition according to the present invention is for intratumoral administration, and/or prepared for intratumoral administration. Administering the composition according to the present invention intratumorally makes it possible that the allogeneic CD4⁺ T cells administered come in contact with a number of alloantigens (for example, a group of MCH class II molecules in a subject), and induce an inflammatory reaction at tumor spots. This makes it possible to elicit the subject's immune response to tumor-cell-derived antigens (for example, tumor antigens in the subject), and consequently enhance the effects of the present invention.

The composition according to the present invention may be combined with another antitumor therapy, for example, at least one of surgical excision, a radiation therapy, a chemotherapy, and other immune cellular therapies.

In one embodiment, the present invention can have an effect of achieving a high treatment and/or prevention effect on a tumor. In one embodiment, the present invention can have an effect of having less or no side effect, such as toxicity (for example, a decrease in a body weight), on a subject.

In one embodiment, the antitumor effect of the present invention is exerted mainly by activating an immune system (CD8⁺ cells and the like) of a subject oneself. Accordingly, in one embodiment, a subject needs no hematopoietic stem cell transplantation, which is typically needed in a graft-versus-tumor effect. In one embodiment, a subject undergoes no hematopoietic stem cell transplantation immediately before administration, during administration, and/or immediately after administration of the composition according to the present invention.

In one embodiment, the present invention can have a treatment and/or prevention effect on a recurrent tumor and/or metastatic tumor. In one embodiment, the composition according to the present invention is for use in treatment and/or prevention of a recurrent tumor and/or metastatic tumor.

In one aspect, the present invention relates to a method of producing the composition described herein, comprising a step of coculturing an allogeneic CD4⁺ T cell(s) *ex vivo* with an antigen-presenting cell(s) derived from a subject or an individual having MHC class II molecules wholly identical or partially identical with MHC class II molecules of the subject. In this aspect, details of the allogeneic CD4⁺ T cell(s), the subject, the antigen-presenting cell(s) derived from the subject or the individual having MHC class II molecules wholly identical or partially identical with the MHC class II molecules of the subject, the coculture, and the like are as described with respect to the composition according to the present invention.

In one aspect, the present invention relates to a method of treatment and/or prevention of a tumor in a subject, comprising administering the allogeneic CD4⁺ T cell(s) or the composition described herein to the subject, wherein the allogeneic CD4⁺ T cell(s) has (a) MHC class II molecules all or part of which are different from MHC class II molecules of the subject, and contains (b) a cell(s) cocultured *ex vivo* with an antigen-presenting cell(s) derived from the subject or an individual having MHC class II molecules wholly identical or partially identical with the MHC class II molecules of the subject. In this aspect, the subject may be a subject in need for the treatment and/or prevention method, and the dose may be an effective amount for the treatment and/or prevention. In this aspect, details of the allogeneic CD4⁺ T cell(s), the subject, the antigen-presenting cell(s) derived from the subject or the individual having MHC class II molecules wholly identical or partially identical with the MHC class II molecules of the subject, the coculture, and the like are as described with respect to the composition according to the present invention.

### Examples

### <Example 1: Effects of allogeneic CD4⁺ T cells which had been cocultured with autologous dendritic cells against malignant melanoma>

### (Materials and methods)

### (1) Mice, cell lines, antibodies (Abs), and reagents

C57BL/6 (H-2^{b}) and BALB/c (H-2^{d}) mice were purchased from CLEA Japan Inc. (Tokyo, Japan). The experimental protocol was approved by the Fukushima Medical University's Committee on Use and Care of Animals. B16F1, the malignant melanoma cell line derived from C57BL/6, was purchased from RIKEN BRC CELL BANK (Ibaraki, Japan). Antibodies used to isolate cells were purchased from BioLegend, Inc. (San Diego, U.S.A.), unless otherwise specified. Microbead-conjugated antibodies and streptavidin were purchased from Miltenyi-Biotech Inc. (Auburn, U.S.A.). Recombinant mouse GM-CSF, recombinant mouse SCF, recombinant mouse IL-4, and recombinant mouse TNFα were purchased from R&D Systems, Inc. (Minneapolis, U.S.A.). Recombinant human IL-2 was purchased from Shenandoah Biotechnology Inc. (Warwick, U.S.A.).

### (2) Harvest of hematopoietic stem cells and generation of dendritic cells

Dendritic cells were generated *in vitro* in accordance with the method reported previously (Mochizuki K. et al., Blood, 2016, 23, 127(25), 3270-80). Briefly, c-kit positive cells isolated from a bone marrow were cultured in an RPMI1640 culture medium containing 10% FBS, recombinant mouse GM-CSF (10 ng/ml), recombinant mouse SCF (10 ng/ml), recombinant mouse IL-4 (2.5 ng/ml), and recombinant mouse TNFα (4 ng/ml). Ten days after the culture, the dendritic cells were stimulated with LPS (100 ng/ml) (Sigma-Aldrich Co. LLC) and R848 (100 ng/ml) (InvivoGen Inc.) for 6 hours. Then, dendritic cells activated were collected.

### (3) Harvest of allogeneic T cells and ex vivo coculture thereof with the dendritic cells in cases where a host is C57BL/6 mice

CD4⁺ T cells and/or CD8⁺ T cells were isolated from spleens of BALB/c mice, using microbead-conjugated antibodies (MiniMACS; Miltenyi Biotech GmbH, Germany). The purity was consistently 92% or more. The isolated T cells were stimulated by coculture with the dendritic cells derived from the host C57BL/6 mice (autologous) and generated *in vitro* as above-mentioned, in a 96-well U-bottom plate, using an RPMI1640 culture medium containing 10% FBS and recombinant human IL-2 (200 U/ml). The coculture was performed under 5% CO₂ at 37°C for three days, and ratios of the T cells and the dendritic cells were 2:1 to 4:1. The resulting T cells were used in the below-mentioned *in vivo* injection.

### (4) Cancer-bearing (malignant melanoma) mouse model

C57BL/6 mice (host) were inoculated with the malignant melanoma cell line B16F1 through subcutaneous injection, on day 0 (the amounts of the tumor inoculated are mentioned below). Then, the *ex-vivo* cocultured allogeneic T cells (2 ×10⁶ cells/mouse) derived from the BALB/c mice and prepared by the above-mentioned coculture, were administered intratumorally through direct injection on the ninth day after the tumor inoculation. States of tumors and whole bodies were observed every two to three days. Diameters of the tumors were measured using calipers, and tumor volumes were determined from the measured values in accordance with the following formula: Tumor volume (mm³) = (major axis × minor axis²)/2. Mice bearing a tumor having a major axis of more than 18 mm were all euthanized for ethical reasons. In addition, for purpose of observing complications, body weights of the mice were measured every two to three days, after administering the *ex-vivo* cocultured allogeneic T cells.

### (Results)

The CD4⁺ T cells isolated from the BALB/c mice (allogeneic) were stimulated by coculture with the dendritic cells derived from the C57BL/6 mice (autologous), and the resulting allogeneic CD4⁺ T cells were administered intratumorally to the C57BL/6 mice which had been inoculated with the tumor (B 16F1). The tumor volume, survival rate, and body weight change rate of the resulting mice are shown in Figures 1 to 3.

As seen in Figure 1 (subcutaneous inoculation with 0.5 × 10⁶ cells of B16F1 on day 0), administration of the allogeneic CD4⁺ T cells decreased the tumor volume markedly. In contrast, administration of the control instead of CD4⁺ T cells and administration of the autologous CD4⁺ T cells (isolated from C57BL/6 mice) did not decrease the tumor volume. In this regard, administration of (allogeneic) CD8⁺ T cells derived from BALB/c mice which had been cocultured with dendritic cells derived from C57BL/6 mice also did not decrease a tumor volume (data not shown). In addition, administration of (allogeneic) CD4⁺ T cells derived from BALB/c mice which had not been cocultured with dendritic cells derived from C57BL/6 mice did not decrease a tumor volume (data not shown).

Additionally, as seen in Figure 2, the mice treated with the allogeneic CD4⁺ T cells exhibited a marked improvement in the survival rate, compared with the control and the mice treated with the autologous CD4⁺ T cells.

In contrast, as seen in Figure 3, no large difference is recognized in the body weight change rate between the mice treated with the allogeneic CD4⁺ T cells and the control. This indicates that administration of the allogeneic CD4⁺ T cells did not induce toxicity accompanied by a change in the body weight.

These results indicate that the allogeneic CD4⁺ T cells exert a marked tumor regression effect without toxicity.

### <Example 2: Effects of allogeneic CD4⁺ T cells when host CD8⁺ cells were depleted>

### (Materials and methods)

C57BL/6 mice were subcutaneously inoculated with 1.0 × 10⁶ cells of B16F1 on day 0, in accordance with the method described in Example 1. Allogeneic CD4⁺ T cells (derived from BALB/c mice) which had been cocultured with dendritic cells derived from C57BL/6 mice (autologous), were prepared in accordance with the method described in Example 1, and administered on day 9. The host CD8⁺ T cells were depleted using anti-mouse CD8a antibodies (which were intraperitoneally injected in an amount of 500 µg on the eighth day from the tumor inoculation, and in an amount of 250 µg each on the 10th day and the 12th day: BioXcel Therapeutics Inc., New Haven, U.S.A.). To the control, IgG (BioXcel Therapeutics Inc., New Haven, U.S.A.) was administered intraperitoneally.

### (Results)

As seen in Figure 4, when the host CD8⁺ cells were depleted by the anti-CD8 antibodies, the tumor regression effect of the allogeneic CD4⁺ T cells was not seen, and tumor was increased.

This result indicates that the tumor regression by the allogeneic CD4⁺ T cells depends on the host CD8⁺ cells.

### <Example 3: A tumor recurrence prevention effect (long-term effect) of allogeneic CD4⁺ T cells>

### (Materials and methods)

C57BL/6 mice were subcutaneously inoculated with 0.5 × 10⁶ cells of B16F1 on day 0, and then treated with allogeneic CD4⁺ T cells (derived from BALB/c mice) which had been cocultured with dendritic cells derived from C57BL/6 mice (autologous), on day 9. After six months, the C57BL/6 mice that survived were inoculated with a tumor (subcutaneously inoculated with 2.5 × 10⁶ cells of B16F1) for the second time, and tumor volumes were measured. Naive C57BL/6 mice, which had not inoculated with the tumor and received cellular therapy in the past, were used as a control. In this regard, the method of preparing the allogeneic CD4⁺ T cells (derived from BALB/c mice) was in accordance with the method described in Example 1.

### (Results)

As seen in Figure 5, tumor development was inhibited even in cases where the allogeneic CD4⁺ T cells which had been cocultured with the dendritic cells derived from C57BL/6 mice were administered to mice, and the mice that survived were inoculated with a tumor again after six months. This result indicates that administering the allogeneic CD4⁺ T cells to a tumor induces tumor-specific immunity in a host, and that the antitumor immunity has a long-term recurrence prevention effect.

### <Example 4: An effect of allogeneic CD4⁺ T cells against a tumor other than a tumor at an administration site>

### (Materials and methods)

Under the same conditions as described in Example 1, the right side abdomen and left side abdomen of C57BL/6 mice were inoculated with the same amount of B16F1 (1.0 × 10⁶ cells each) by subcutaneous injection, and then, allogeneic CD4⁺ T cells (derived from BALB/c mice) (2 × 10⁶ cells/mouse) which had been cocultured with the dendritic cells derived from C57BL/6 mice were administered into the tumor of the left side abdomen by injection.

### (Results)

The tumor volume is shown in Figure 6, and the survival rate is shown in Figure 7. As seen in Figure 6 (A to E: control, F to J: administration), the tumor regressed not only in the left side abdomen (the administration site) but also in the right side abdomen in four mice out of the five mice treated with the allogeneic CD4⁺ T cells. Additionally, as seen in Figure 7, the mice treated with the allogeneic CD4⁺ T cells exhibited a marked increase in the survival rate. These results indicate that administration of the allogeneic CD4⁺ T cells can induce systemic antitumor immunity in a host, and achieve an effect against a tumor other than a tumor at the administration site.

### <Example 5: An effect of allogeneic CD4⁺ T cells which had been cocultured with dendritic cells derived from an individual having the same MHC class II molecules as a host, against a tumor>

### (Purpose)

It is verified whether a tumor regression effect is seen when using dendritic cells from different individuals having the same MHC class II molecules.

### (Materials and methods)

129X1 (H-2^{b}) mice were purchased from Japan SLC, Inc. (Shizuoka, Japan). CD4⁺ T cells isolated from BALB/c mice were cocultured with dendritic cells derived from C57BL/6 or 129X1 mice (having the same MHC class II molecules as C57BL/6 mice), and the obtained allogeneic CD4⁺ T cells were administered intratumorally to C57BL/6 mice which had been inoculated with a tumor (B16F1), and tumor volumes were measured. The other methods are as described in Example 1.

### (Results)

As seen in Figure 8 (subcutaneous inoculation with 1.0 × 10⁶ cells of B16F1 on day 0), administration of the allogeneic CD4⁺ T cells which had been cocultured with the dendritic cells derived from the 129X1 mice decreased the tumor volume markedly (Figures 8B and 8C) in the same manner as administration of the allogeneic CD4⁺ T cells which had been cocultured with the dendritic cells derived from the C57BL/6 mice. In contrast, administration of the control instead of CD4⁺ T cells did not decrease the tumor volume (Figure 8A).

These results indicate that allogeneic CD4⁺ T cells which had been cocultured with dendritic cells derived from an individual having the same MHC class II molecules as a host achieve a marked tumor regression effect in the same manner as the allogeneic CD4⁺ T cells activated by the autologous dendritic cells.

### <Example 6: An effect of allogeneic CD4⁺ T cells for which cell sources were frozen and allogeneic CD4⁺ T cells for which a cell product was frozen, against a tumor>

### (Materials and methods)

STEM-CELLBANKER (registered trademark) DMSO Free GMP grade (Zenoaq Resource Co., Ltd., Fukushima, Japan) was used as a cell preservation solution. In the cell source freezing, cell sources were cryopreserved using STEM-CELLBANKER (registered trademark) DMSO FreeGMP grade (Zenoaq Resource Co., Ltd.) at -80°C at the stage of being c-kit-positive cells isolated from bone marrows of C57BL/6 mice and at the stage of being CD4⁺ T cells isolated from BALB/c mice. The cell sources were thawed when used. The other methods are as described in Example 1.

In the cell product freezing, CD4⁺ T cells (a cell product) which had been cocultured with the dendritic cells derived from C57BL/6 mice in accordance with the method described in Example 1 were prepared, and cryopreserved using STEM-CELLBANKER (registered trademark) DMSO Free GMP grade (Zenoaq Resource Co., Ltd.) at -80°C. Before administration, the cell product was thawed and centrifuged, and refloated in PBS (phosphate buffered saline) to use for an *in vivo* injection.

### (Results)

As seen in Figure 9 (subcutaneous inoculation with 1.0 × 10⁶ cells of B16F1 on day 0), even in cases where the cell sources (the c-kit positive cells and the CD4⁺ T cells before coculture with the dendritic cells) or the cell product (the CD4⁺ T cells obtained by coculture with the dendritic cells) were (was) cryopreserved, administration of the allogeneic CD4⁺ T cells decreased the tumor volume markedly in the same manner as in cases where the cell sources or the cell product were (was) not cryopreserved (Figures 9B to 9D). In contrast, administration of the control instead of CD4⁺ T cells did not decrease the tumor volume (Figure 9A).

These results indicate that allogeneic CD4⁺ T cells do not lose the tumor regression effect even in cases where cell sources or a cell product are (is) cryopreserved.

### <Example 7: Effects of allogeneic CD4⁺ T cells which had been cocultured with autologous dendritic cell, against another tumor (colonic cancer)>

### (Materials and methods)

Colon-26, colonic cancer cell lines derived from BALB/c, were obtained from Cell Resource Center for Biomedical Research, Institute of Development, Aging and Cancer, Tohoku University (Sendai, Japan).

The host was BALB/c mice, the cancer cell line inoculated was Colon-26 (5 × 10⁴ cells/mouse), the allogeneic CD4⁺ T cells administered were CD4⁺ T cells isolated from C57BL/6 mice which had been cocultured with dendritic cells isolated from BALB/c mice. The other methods are as described in Example 1.

### (Results)

CD4⁺ T cells isolated from C57BL/6 mice were cocultured with dendritic cells derived from BALB/c mice, and the resulting allogeneic CD4⁺ T cells were administered intratumorally to BALB/c mice which had been inoculated with a tumor (Colon-26). Tumor volumes and survival rates in this case are shown in Figures 10 and 11, respectively.

As seen in Figure 10 (subcutaneous inoculation with 5 × 10⁴ cells of Colon-26 on day 0), the mice which received the allogeneic CD4⁺ T cells exhibited a marked decrease in an increase rate of the tumor volume, compared with the control non-treated with CD4⁺ T cells.

Additionally, as seen in Figure 11, the mice treated with the allogeneic CD4⁺ T cells exhibited an improvement in the survival rate compared with the mice treated with the control.

These results indicate that the allogeneic CD4⁺ T cells can also achieve a marked tumor regression effect against malignant tumors other than a melanoma. In addition, the results indicate that the effects of the present invention are not accidentally exerted by a combination of an antigen-presenting cell derived from a specific species of mouse and a CD4⁺ T cell derived from a specific species of mouse.

All the publications, patents, and patent applications cited herein are incorporated herein by reference in their entirety.

## Claims

1. A composition for use in treatment and/or prevention of a tumor in a subject comprising an allogeneic CD4⁺ T cell,
wherein the allogeneic CD4⁺ T cell has (a) MHC class II molecules all or part of which are different from MHC class II molecules of the subject, and contains (b) a cell cocultured *ex vivo* with an antigen-presenting cell derived from the subject or an individual having MHC class II molecules wholly identical or partially identical with the MHC class II molecules of the subject.

2. The composition according to claim 1, wherein the antigen-presenting cell is a dendritic cell.

3. The composition according to claim 1 or 2, for use in intratumoral administration.

4. A method of producing the composition according to any one of claims 1 to 3, comprising the step of coculturing an allogeneic CD4⁺ T cell *ex vivo* with an antigen-presenting cell derived from the subject or an individual having MHC class II molecules wholly identical or partially identical with the MHC class II molecules of the subject.
